Europäisches Patentamt

European Patent Office

Office européen des brevets

(11) Veröffentlichungsnummer: **0 341 483**

**A2**

(12) # EUROPÄISCHE PATENTANMELDUNG

(21) Anmeldenummer: 89107440.3

(51) Int. Cl.⁴: **G01N 21/64 , A61B 5/10**

(22) Anmeldetag: 25.04.89

(30) Priorität: 07.05.88 DE 3815743

(43) Veröffentlichungstag der Anmeldung:
**15.11.89 Patentblatt 89/46**

(84) Benannte Vertragsstaaten:
**AT CH FR GB IT LI NL SE**

(71) Anmelder: **Firma Carl Zeiss**

**D-7920 Heidenheim (Brenz)(DE)**

(84) **CH FR IT LI NL SE AT**

Anmelder: **CARL-ZEISS-STIFTUNG trading as CARL ZEISS**

**D-7920 Heidenheim (Brenz)(DE)**

(84) **GB**

(72) Erfinder: **Schwarz, Jürgen, Dr.**
**Am Haselstrauch 1**
**D-7082 Oberkochen(DE)**
Erfinder: **Lohmann, Wolfgang, Prof. Dr.**
**Petersweiher 20**
**D-6300 Giessen(DE)**

(54) **Vorrichtung zur Messung und Auswertung von Eigenfluoreszenzspektren organischer Gewebeflächen.**

(57) Ein modifiziertes Axiotron ermöglicht die Aufnahme und Auswertung von Eigenfluoreszenzspektren organischer Gewebeflächen mit dem Vorteil einer koaxial zur Beobachtungsrichtung verlaufenden Ausleuchtung des Objektes.

Fig. 4

EP 0 341 483 A2

## Vorrichtung zur Messung und Auswertung von Eigenfluoreszenzspektren organischer Gewebeflächen

Die Erfindung betrifft eine Vorrichtung zur Messung und Auswertung von Eigenfluoreszenzspektren organischer Gewebeflächen nach dem Oberbegriff des Patentanspruches 1.

Eine derartige Vorrichtung ist aus der DE 35 42 167 A1 im Zusammenhang mit der nicht vorveröffentlichten Patentanmeldung P 37 18 202.1 bekannt.

Die bekannte Vorrichtung verwendet als Lichtquelle die Projektion eines Spaltbildes auf die zu untersuchende organische Gewebefläche.

Der vorliegenden Erfindung liegt die Aufgabe zugrunde, für den genannten Zweck eine Vorrichtung anzugeben, die eine flächenhafte Ausleuchtung des Untersuchungsobjektes erlaubt.

Diese Aufgabe wird erfindungsgemäß mit einer Vorrichtung nach den kennzeichnenden Merkmalen des Patentanspruches 1 gelöst. Weitere Ausgestaltungen der Erfindung ergeben sich aus den Merkmalen der Unteransprüche.

Die mit der Erfindung erzielten Vorteile bestehen insbesondere darin, daß modulartige Teile eines bekannten Gerätes - eines unter dem Namen Axiotron bekannten Mikroskops - verwendet werden können, wodurch Kosten für eine neue Produktionsserie einsparbar sind. Ein weiterer Vorteil der Erfindung liegt in der koaxial zur Beobachtungsrichtung verlaufenden Beleuchtung des Objekts.

Ein Ausführungsbeispiel der Erfindung ist in der Zeichnung dargestellt und wird im folgenden näher beschrieben.

Es zeigen

Figur 1 ein Mikroskop-Photometer bekannter Bauart, das unter dem Namen Axiotron auf dem Markt ist;

Figur 2 ein erfindungsgemäß modifiziertes Axiotron im Teilschnitt von der Seite gesehen;

Figur 3 ein an einem Bodenstativ befestigtes modifiziertes Axiotron;

Figur 4 den optischen Strahlengang in einem erfindungsgemäß modifizierten Axiotron.

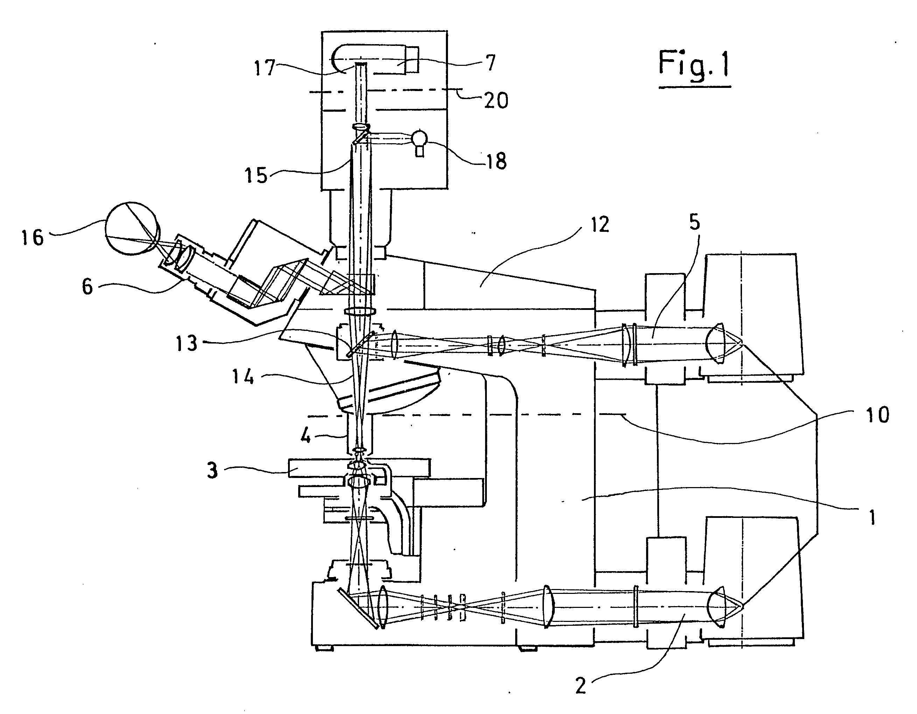

In der Darstellung des in Figur 1 gezeigten Axiotron ist mit dem Bezugszeichen (1) das Mikroskopstativ bezeichnet. Auf die Durchlichtbeleuchtungsvorrichtung verweist das Bezugszeichen (2), und auf den Okulartubus das Bezugszeichen (6). Mit (16) ist ein Beobachterauge gekennzeichnet. Dem Objekttisch ist das Bezugszeichen (3) und dem Objektiv das Bezugszeichen (4) zugeordnet. Mit der Kennziffer (5) ist eine Auflichtbeleuchtungsvorrichtung gekennzeichnet, deren Beleuchtungsstrahlen über eine Teilerplatte (13) koaxial in den Beobachtungsstrahlengang (14) des Axiotron eingespiegelt wird. Mit (15) ist eine in einer Zwischenbildebene angeordnete Photometermeßblende bezeichnet und mit (18) eine Hilfslichtquelle zum Beleuchten dieser Meßblende. Das Bezugszeichen (17) bezieht sich auf die Pupillenebene einer Photokathode (7). An dem gezeigten Axiotron ist mit dem Bezugszeichen (10) eine erste Schnittstelle angedeutet, welche das Stativ (1) und den Objektivtubus an der bezeichneten Stelle mechanisch so durchtrennt, daß ein zur Messung und Auswertung von Eigenfluoreszenzspektren organischer Gewebeflächen geeignetes Objektiv eingesetzt werden kann. Eine zweite Schnittstelle ist mit dem Bezugszeichen (20) gekennzeichnet und soll den Ansatz eines Spektrographen oder eines Lichtleiter-Querschnittswandlers, der ein kreisförmiges Gesichtsfeld in ein rechteckiges umwandelt und zu einem Spektrographen führt, ermöglichen.

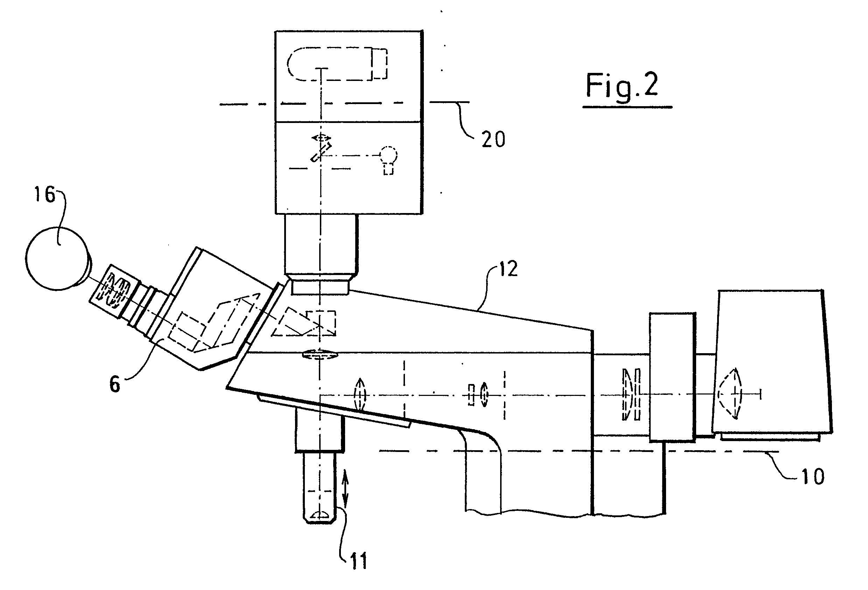

In der Darstellung der Figur 2 ist der Rumpf (12) des Axiotron gezeigt, der nach Abtrennen des unterhalb der Schnittstelle (10) liegenden Teiles und des oberhalb der Schnittstelle (20) liegenden Teiles verbleibt.

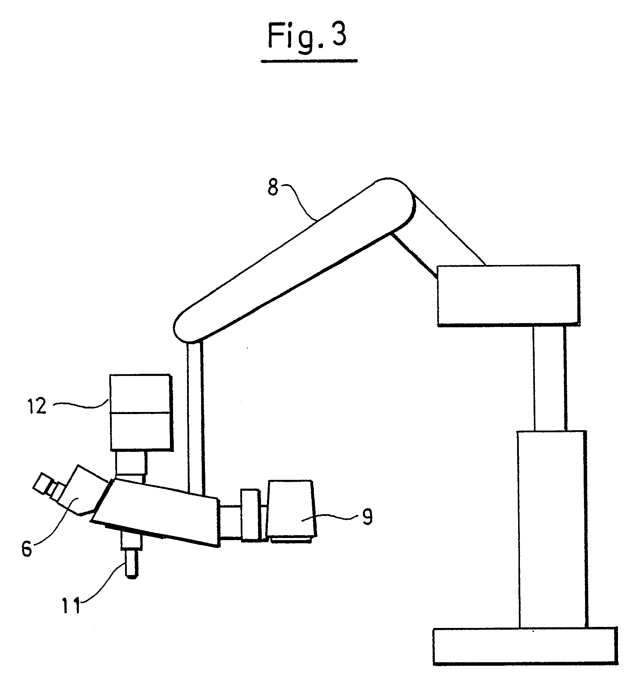

In der Darstellung der Figur 3 ist die Befestigung des Rumpfes (12) an einem Bodenstativ (8) gezeigt, das einen freibeweglichen Arm aufweist und mit einer Kipp- und Schwenkvorrichtung ausgerüstet ist. Dieses Bodenstativ ist an sich bekannt. Anstelle eines Bodenstatives kann auch ein geeignetes bekanntes Wand- oder Deckenstativ verwendet werden.

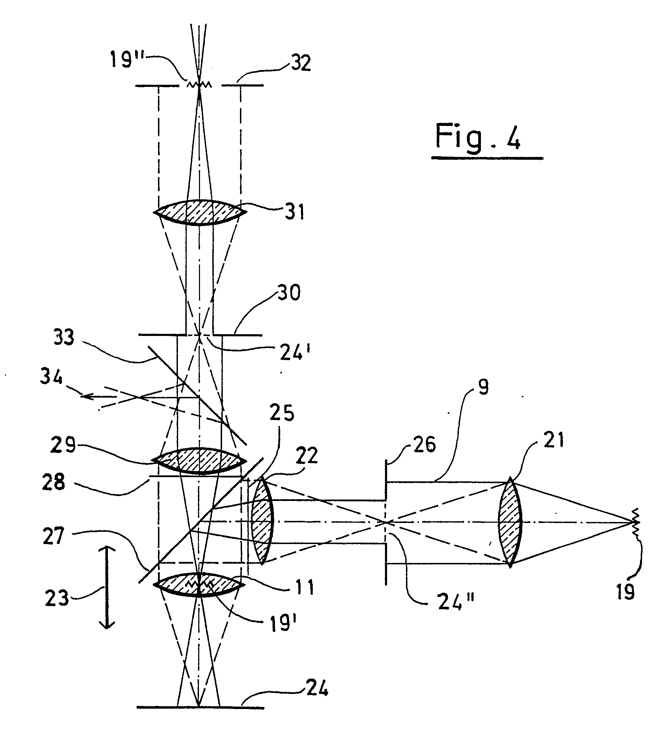

In der Darstellung der Figur 4 ist mit 19 eine zur Aufnahme von Eigenfluoreszenzspektren geeignete Lichtquelle bekannter Bauart bezeichnet. Es kann sich beispielsweise um eine HBO-Lampe handeln. Mit (21) und (22) sind Kondensorlinsen zur Abbildung der Lichtquelle (19) in die Ebene 19′ des Objektives (11) gekennzeichnet. Das Objektiv (11) ist, wie mit Doppelpfeil (23) angedeutet, in vertikaler Richtung beweglich und fokussierbar. Geeignete Fokussiermechanismen sind beispielsweise in der US-PS 4 342 905 und in dem Aufsatz "Laser-Scanning-Mikroskop mit automatischer Fokussierung" in MICROSCOPICA ACTA, Vol. 87, Nr. 2, March 1983, Seiten 129-138 beschrieben. Mit (24) ist die Objektfläche bezeichnet, die eine remittierende und fluoreszierende Oberfläche aufweist. Im Beleuchtungsstrahlengang ist mit (25) ein Fluoreszenz-Anregungsfilter und mit (26) eine Leuchtfeldblende, die in einer Bildebene (24′′) des Objektes (24) angeordnet ist, bezeichnet. Durch einen Farbteiler (27) gelangt das vom Objekt aus gesandte Fluoreszenzlicht über ein in den Strahlengang ein- und ausschwenkbares Sperrfilter (28) und die Tubuslinse (29) durch eine in der ersten Objektbildebene (24′) angeordnete Meßblende (30) über eine Relaislinse (31) in den Eintrittsspalt (32) eines in der Fig. 4 nicht eingezeichneten Spektrographen oder zu einem Lichtleiter-Querschnittswandler, der zu einem Spektrographen führt. Der Eintrittsspalt (32) ist in der zweiten Bildebene (19″) der Lichtquelle (19) angeordnet. Über einen schaltbaren Spiegel (33) kann vom Objekt ausgesendetes Licht in eine in Pfeilrichtung (34) liegende Beobachtungsvorrichtung, beispielsweise einen Okulartubus gelangen.

## Ansprüche

1. Vorrichtung zur Messung und Auswertung von Eigenfluoreszenzsprektren organischer Gewebeflachen, wobei die Anregungswellenlänge $\lambda_A$ zwischen 320nm und 550nm liegt, das Spektralphotometer das rückgestreute Licht im Wellenlängenbereich zwischen 320nm und 700nm aufnimmt und die Auswerteeinrichtung die Maximalintensität des reflektierten Lichtes bei der Wellenlänge $\lambda_A$ und die Maximalintensität des Fluoreszenzlichtes im zu $\lambda_A$ längerwelligen Bereich ermittelt, dadurch gekennzeichnet, daß ein an sich bekanntes Mikroskopphotometer (Figur 1) an seinem Stativ (1) an einer ersten Schnittstelle (10) so durchtrennt wird, daß der untere Teil des Mikroskopphotometers, bestehend aus Durchlichtbeleuchtung (2), Objekttisch (3) und Objektiv (4) entfernt ist, daß der obere Teil des Mikroskopphotometers, bestehend aus koaxialer Auflichtbeleuchtung (5), Okulartubus (6) und Photokathode (7) eine zweite Schnittstelle (20) in der Pupillenebene der Photokathode (7) aufweist, daß der so entstandene Rumpf (12) des Mikroskop-Photometers an einem schwenkbaren Statiy (8) befestigt und mit einem UV-tüchtigen und fokussierbaren Objektiv (11), einer Beleuchtungseinrichtung (9) zur Fluoreszenzanregung der untersuchten organischen Gewebeflache und einem an der zweiten Schnittstelle (20) vorgesehenen Spektrographen ausgerüstet ist und daß die Anregungswellenlänge zwischen 250nm und 550nm wählbar und das rückgestreute Licht zwischen 250 und 700nm erfaßbar ist.

2. Vorrichtung nach Ansruch 1, dadurch gekennzeichnet, daß anstelle des Spektrographen ein Lichtleiter-Querschnittswandler vorgesehen ist, der zu einem Spektrographen führt.

3. Vorrichtung nach Anspruch 1, dadurch gekennzeichnet, daß anstelle des Objektivs (11) ein (starres) Endoskop vorgesehen ist.

EP 0 341 483 A2

Fig.1

Fig.2

EP 0 341 483 A2

# Fig.3

88073 P Ef

Fig. 4

88 013 PEP